(19) Europäisches Patentamt
European Patent Office
Office européen des brevets

(11) Publication number: **0 293 053 B1**

(12) # EUROPEAN PATENT SPECIFICATION

(45) Date of publication of patent specification: **04.03.92**

(51) Int. Cl.5: **C07C 51/56**, C07C 55/02

(21) Application number: **88201056.4**

(22) Date of filing: **25.05.88**

(54) **Process for the preparation of cyclic carboxylic anhydrides.**

(30) Priority: **27.05.87 GB 8712455**

(43) Date of publication of application:
**30.11.88 Bulletin 88/48**

(45) Publication of the grant of the patent:
**04.03.92 Bulletin 92/10**

(84) Designated Contracting States:
**BE DE FR GB IT NL**

(56) References cited:
**EP-A- 0 194 707**
**EP-A- 0 235 864**
**US-A- 4 414 409**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Drent, Eit**
**Badhuisweg 3**
**NL-1031 CM Amsterdam(NL)**

EP 0 293 053 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process for the preparation of cyclic anhydrides of optionally substituted alkanedioic acids having 4 or 5 carbon atoms in the ring or of a mixture of such cyclic anhydrides.

Such cyclic anhydrides may be prepared as described in Bull. Soc. Chim. Fr. 1975, (9-10, Pt. 2), 2189-2194, by acid hydrolysis and decarboxylation of diethyl beta-alkyl-alpha-cyanosuccinate, the alkyl group being an ethyl or a propyl group, to give monosubstituted acids, $HO_2CCH=CH_2CH(R)CO_2H$, R being an ethyl or a propyl group, which were transformed to the anhydrides by heating. This known process for the preparation of cyclic anhydrides requires a starting material which is difficultly accessible.

It is an object of the present invention to prepare the cyclic anhydrides mentioned hereinbefore in a simple manner from simple starting compounds and with high selectivity.

Accordingly, the invention provides a process for the preparation of cyclic anhydrides of optionally substituted alkanedioic acids having 4 or 5 carbon atoms in the ring or of a mixture of such cyclic anhydrides which process comprises reacting an optionally substituted alkenoic acid with carbon monoxide in the presence of a catalytic system obtainable by combining the following components:-

component (a) - a palladium compound,
component (b) - a phosphine, arsine and/or stibine, and
component (c) - a protonic acid having a $pK_a$, measured at 25 °C in aqueous solution, of less than 2.

The process according to the present invention is carried out in the liquid phase in which the palladium compound may be heterogeneous but is preferably homogeneous. Suitable homogeneous palladium compounds are the salts of palladium(II) with, for instance, nitric acid, sulphuric acid, or, which is preferred, alkanoic acids having not more than 12 carbon atoms per molecule. A palladium compound used by preference is palladium(II) acetate. Other examples of palladium(II) compounds are palladium(II) formate and palladium(II) propanoate. Salts of hydrohalogenic acids may also be used. Moreover, palladium complexes may be used, for instance palladium acetylacetonate, tetrakistriphenylphosphinepalladium, bis-tri-o-tolylphoshinepalladium acetate or bis-triphenylphosphinepalladium sulphate. A mixture of palladium compounds may be used as component (a).

It has, surprisingly, been found that the process according to the present invention allows the preparation of cyclic anhydrides of optionally substituted alkanedioic acids having four carbon atoms in the ring with an extremely high selectivity. The selectivity to a certain compound, expressed in a percentage, is defined herein as 100 x p:q in which "p" is the amount of starting compound that has been converted into that certain compound and "q" is the total amount of starting compound that has been converted. Therefore, according to a preferred embodiment of the present invention compound (b) is a chelating ligand comprising an organic compound containing as coordinating atoms at least two atoms of phosphorus, arsenic or antimony which are connected through a divalent organic bridging group having at least two carbon atoms in the bridge. For example, by this preferred embodiment 3-pentenoic acid can be converted quantitatively into ethylsuccinic anhydride. The two or more coordinating atoms may be the same or different, for example two arsenic atoms, a phosphorus or an arsenic atom, or an arsenic and an antimony atom. The chelating ligand preferably has the general formula I

$$R^1 - \underset{R^2}{\underset{|}{P}} - R^3 - \underset{R^4}{\underset{|}{P}} - R^5 \qquad (I)$$

in which $R^1$, $R^2$, $R^4$ and $R^5$ represent identical or different optionally substituted hydrocarbon groups and $R^3$ represents a chain consisting of two to six optionally substituted methylene groups. Any substituents present in the chelating ligand preferably do not cause steric hindrance to the formation of complex compounds with the palladium(II) compound.

Hydrocarbon groups $R^1$, $R^2$, $R^4$ and $R^5$ will as a rule contain 2 to 18 carbon atoms, preferably 6 to 14 carbon atoms. Aryl groups are the most suitable, in particular the phenyl group. Preferred bridging groups $-R^3-$ are those having the formula $-(CR^6R^7)_n$ in which $R^6$ and $R^7$ are hydrogen atoms or optionally substituted hydrocarbon groups preferably offering no steric hindrance and n is an integer of at least two, preferably not more than 5, and most preferably 2, 3 or 4. Substituents $R^6$ and $R^7$ are preferably hydrogen atoms. The bridging groups $R^3$ may also make part of a cyclic structure, e.g. an aromatic or cycloaliphatic group, the carbon to carbon bond or bonds in the bridge may be saturated or unsaturated and in the bridge or in the cyclic or non-cyclic groups attached to the bridge one or more hetero atoms, e.g. sulphur, oxygen,

iron or nitrogen atoms, may replace carbon atoms, other than the two carbon atoms which must be present in the bridge linking both phosphorus atoms.

Examples of suitable chelating ligands are
1,3-di(diphenylphosphino)propane,
1,4-di(diphenylphosphino)butane,
2,3-dimethyl-1,4-di(diphenylphosphino)butane,
1,5-di(methylphenylphosphino)pentane,
1,4-di(dicyclohexylphosphino)butane,
1,5-di(dinaphthylphosphino)pentane,
1,3-di(di-p-tolylphosphino)propane,
1,4-di(di-p-methoxyphenylphosphino)butane,
1,2-di(diphenylphosphino)ethene,
2,3-di(diphenylphosphino)-2-butene,
1,3-di(diphenylphosphino)-2-oxopropane,
2-methyl-2-(methyldiphenylphosphino)-1,3-di(diphenylphosphino)propane,
o,o′-di(diphenylphosphino)biphenyl,
1,2-di(diphenylphosphino)benzene,
2,3-di(diphenylphosphino)naphthalene,
1,2-di(diphenylphosphino)cyclohexane,
2,2-dimethyl-4,5-di(diphenylphosphino)dioxolane and

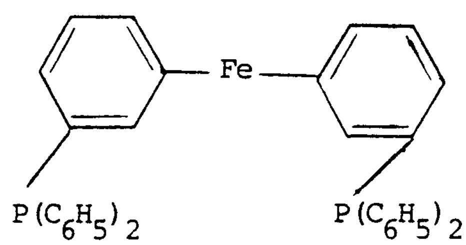

Very good results have been obtained with 1,4-di(diphenylphosphino)butane. A mixture of chelating ligands of the general formula I may be used.

Furthermore, it has surprisingly been found that the process according to the present invention allows the preparation of a mixture of a cyclic anhydride of an optionally substituted alkanedioic acid having 4 carbon atoms in the ring and a cyclic anhydride of an optionally substituted alkanedioic acid having 5 carbon atoms in the ring with an extremely high selectivity to the total of the two anhydrides. Therefore, according to a preferred embodiment of the present invention compound (b) has the general formula II

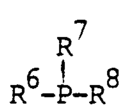 (II)

in which $R^6$, $R^7$ and $R^8$ each individually represent an optionally substituted aryl group. For example, by this preferred embodiment 3-pentenoic acid can be converted quantitatively into a mixture of ethylsuccinic anhydride and 2-methylglutaric anhydride.

The substituted or unsubstituted aryl groups $R^6$, $R^7$ and $R^8$ of the phosphine of the general formula II preferably contain not more than 18, in particular in the range of from 6 to 14 carbon atoms. Examples of suitable $R^6$, $R^7$ and $R^8$ groups are the naphthyl group and in particular, the phenyl group. Suitable substituents are halogen atoms and alkyl, aryl, alkoxy, carboxy, carbalkoxy, acyl, trihalogenmethyl, cyano, dialkylamino, sulphonylalkyl and alkanoyloxy groups.

Examples of suitable phosphines are tri-p-tolylphosphine, tri(p-chlorophenyl)phosphine, tri-p-methoxyphenylphosphine, o-diphenylphosphinobenzoic acid and in particular triphenylphosphine. A mixture of phosphines of the general formula II may be used.

A mixture of chelating ligands of the general formula I and phosphines of the general formula II may be used as component (b). In this case cyclic anhydrides of optionally substituted alkanedioic acids having four carbon atoms in the ring are usually formed with an extremely high selectivity.

The protonic acid having a $pK_a$ of less than 2 preferably has an anion which is non-coordinating, by

which is meant that little or no covalent interaction takes place between the palladium and the anion of the protonic acid.

A preferred group of acids has the general formula III

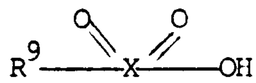

(III)

in which X represents a sulphur or a chlorine atom and, if X represents a chlorine atom, $R^9$ represents an oxygen atom and, if X represents a sulphur atom, $R^9$ represents an OH group or an optionally substituted hydrocarbon group.

When the hereinbefore stated acids are used in the process according to the invention, the anions of the acids can be considered to be non-coordinating.

In the acids having the general formula III, the optionally substituted hydrocarbon group represented by $R^9$ is preferably an alkyl, aryl, aralkyl or alkaryl group having 1-30, in particular 1-14, carbon atoms. The hydrocarbon group may, for example, be substituted with halogen atoms, in particular fluorine atoms. Preferred acids of the general formula III are perchloric acid, sulphuric acid, p-toluenesulphonic acid and trifluoromethanesulphonic acid. p-Toluenesulphonic acid is particularly preferred. Another suitable acid is 2-hydroxypropane-2-sulphonic acid. The acid of the general formula III can also be an ion exchanger containing sulphonic acid groups, such as, for example, Amberlite 252 H ("Amberlite" is a trade name). In that case, the hydrocarbon group $R^9$ is a polymeric hydrocarbon group, for example a polystyrene group substituted with sulphonic acid groups. Further examples of suitable acids are those that can be formed, possibly in situ, by interacting a Lewis acid such as, for example $BF_3$, $AsF_5$, $SbF_5$, $PF_5$, $TaF_5$ or $NbF_5$ with a Broensted acid such as, for example, a hydrohalogenic acid, in particular HF, fluorosulphonic acid, phosphoric acid or sulphuric acid. Specific examples of acids of the latter type are $H_2SiF_6$, $HBF_4$, $HPF_6$ and $HSbF_6$. Examples of suitable sulphonic acids are fluorosulphonic acid and chloro- sulphonic acid. Other examples of suitable acids are trichloroacetic acid, trifluoroacetic acid, dichloroacetic acid and difluoroacetic acid. A mixture of protonic acids having a $pK_a$ of less than 2 may be used as component (c).

The process according to the invention is suitably carried out in the presence of a solvent, which is preferably aprotic. Examples of such solvents are hydrocarbons, such as hexane, heptane, octane, benzene, toluene, the three xylenes, ethylbenzene, cumene, cyclohexane and decalin; halogenated hydrocarbons, such as dichloromethane, chloroform, 1,2-dichloroethane, perfluoroalkanes, chlorobenzene and the three dichlorobenzenes; sulphones such as diethyl sulphone, diisopropyl sulphone and tetrahydrothiophene 1,1-dioxide (also referred to as "sulfolane"); N,N-dialkyl-substituted amides such as N,N-dimethylformamide and N-methylpyrrolidone; esters such as methyl benzoate, ethyl acetate and amyl acetate; ethers such as diethyl ether, 3,6-dioxaoctane, methyl tert.-butylether, tetrahydrofuran, diisopropyl ether, 1,4-dioxane, 2,5,8-trioxanonane (also referred to as "diglyme"), diphenyl ether and anisole. Very good results have been obtained with ethers.

The following molar ratios are not critical and may vary within wide ranges:

1) the molar ratio of the palladium compound to the optionally substituted alkenoic acid having a chain of at least 3 aliphatic carbon atoms,

2) the molar ratio of component (b) to component (a), and

3) the molar ratio of component (c) to component (a).

The molar ratio sub (1) is usually in the range of from 1:10 to 1:10,000 and preferably from 1:50 to 1:1000, the molar ratios sub (2) and (3) are usually in the range of from 0.5 to 200 and preferably from 1 to 20.

The process according to the present invention can be carried out at a temperature and a pressure which are not critical and which may vary within wide ranges, the temperature suitably in the range of from 20 °C to 250 °C and preferably from 50 °C to 200 °C and the pressure suitably in the range of from 1 to 100 bar and preferably from 20 to 75 bar.

In the process according to the invention the carbon monoxide may be used pure or diluted with an inert gas, such as nitrogen, noble gases or carbon dioxide. Generally the presence of more than 10 %v of hydrogen is undesirable, since under the reaction conditions it may cause hydrogenation of the olefinic compound. Generally preference is given to the use of carbon monoxide or a carbon monoxide-containing gas which contains less than 5 %v of hydrogen.

The process according to the present invention may be carried out using a great variety of optionally substituted alkenoic acids having a chain of at least three aliphatic carbon atoms. A terminal carbon atom of

this chain is the carbon atom of the carbonyl group of the acid. The process may schematically be represented by means of equation A in case a 3-alkenoic acid is used as a starting acid:

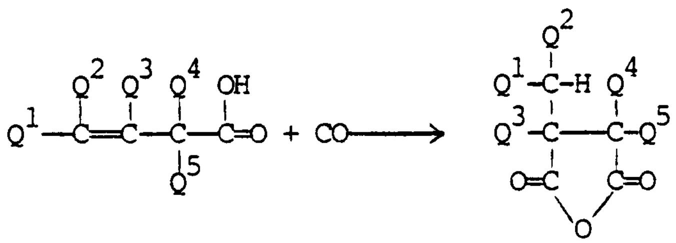

(A)

in which $Q^1$, $Q^2$, $Q^3$, $Q^4$ and $Q^5$ each represent a hydrogen atom or an optionally substituted hydrocarbon group, in which case a cyclic anhydride of an optionally substituted alkanedioic acid having 4 carbon atoms in the ring is formed.

A mixture of cyclic anhydrides is formed when the following reaction B takes place simultaneously with reaction A:

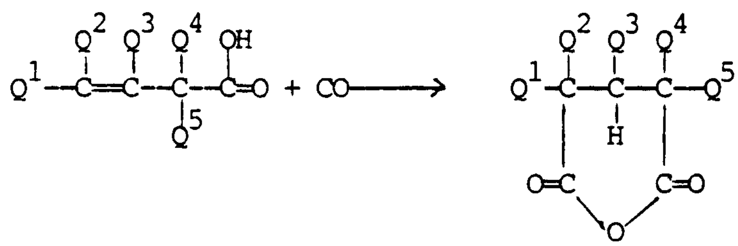

(B)

in which $Q^1$, $Q^2$, $Q^3$, $Q^4$ and $Q^5$ have the same meaning as in equation A.

A hydrocarbon group represented by $Q^1$, $Q^2$, $Q^3$, $Q^4$ and $Q^5$ may be an alkyl group, preferably having in the range of from 1 to 30 carbon atoms, a cycloalkyl group, preferably having in the range of from 3 to 8 carbon atoms, or an aryl group, preferably a phenyl group. $Q^2$, $Q^3$, $Q^4$ and $Q^5$ are most preferably alkyl groups having in the range of from 1 to 5 carbon atoms. Among these alkyl groups methyl and ethyl groups are preferred, in particular methyl groups. However, $Q^2$, $Q^3$, $Q^4$ and $Q^5$ most preferably represent hydrogen atoms.

$Q^1$ in particular represents an alkyl group having in the range of from 1 to 30 carbon atoms. The alkenoic acid is preferably a 3-alkenoic acid and in particular 3-pentenoic acid. Other examples of such acids are 3-butenoic acid, 2-methyl-3-butenoic acid, 2,2-dimethyl-3-butenoic acid, 2-methyl-3-pentenoic acid, 3-hexenoic acid and 2,2-dimethyl-3-hexenoic acid.

Alternatively, the starting alkenoic acid may be a 2-alkenoic acid. For example, crotonic acid is converted into methylsuccinic anhydride, acrylic acid is converted into succinic anhydride and 2-methylcrotonic acid into 1,2-dimethylsuccinic anhydride.

It has, furthermore, surprisingly been found that starting p-alkenoic acids in which p is an integer greater than 3 are isomerized in situ by shifting of the carbon carbon double bond to 3-alkenoic acids under influence of the catalytic system. The 3-alkenoic acids, in turn, are converted into cyclic anhydrides according to the above mentioned equation (A) and, eventually, (B). For example, 10-undecenoic acid is isomerized to 3-undecenoic acid, which, in turn, is converted with extremely high selectivity into octylsuccinic anhydride. Other examples of such starting alkenoic acids are 4-pentenoic acid, 4-hexenoic acid, 5-hexenoic acid, 7-octenoic acid and 9-decenoic acid.

Any hydrocarbon group represented by $Q^1$, $Q^2$, $Q^3$, $Q^4$ and $Q^5$ may, optionally, carry substituents which are inert under the reaction conditions, for example alkoxy groups, preferably those having in the range of from 1 to 5 carbon atoms, or halogen atoms, for example chlorine or bromine atoms.

The process according to the present invention may be carried out batchwise, semi-continuously or continuously. When operating batchwise, the catalytic system, the starting alkenoic acid and the solvent are charged to a reactor to form a liquid phase therein, the reactor is pressurized with carbon monoxide and heated to the desired temperature. When operating continuously, the liquid components can be charged to the reactor continuously to form a liquid phase therein and the carbon monoxide continuously introduced into the reactor to contact the liquid phase containing the catalyst. The gaseous reactants can be withdrawn

from the reactor as a separate effluent, cooled, depressurized and the carbon monoxide can be recycled for further contacting.

The cyclic anhydrides may be isolated from the reaction mixture in any suitable manner, for example by means of extraction or by distillation, obtaining a distillate fraction containing the cyclic anhydrides, and a bottom fraction containing the catalytic system. Suitably, a solvent is chosen that substantially remains in the bottom fraction. Preferably, at least a portion of the bottom fraction containing solvent and catalytic system is re-used in the process according to the invention.

The following Examples further illustrate the invention.

## EXAMPLE 1

A 300 ml magnetically stirred Hastelloy C autoclave ("Hastelloy" is a trade mark) was charged with the following materials:-

anisole 40 ml
3-pentenoic acid 20 ml (200 mmol)
palladium(II) acetate 0.4 mmol
1,4-di(diphenylphosphino)butane 0.8 mmol
tri(p-chlorophenyl)phosphine 2 mmol
p-toluenesulphonic acid 3 mmol

Then, the autoclave was flushed with carbon monoxide, pressurized with carbon monoxide until a partial pressure of 40 bar was obtained, heated to a temperature of 90 °C and kept at this temperature for 2 h. At the end of this period the autoclave was allowed to adopt ambient temperature and the contents thereof were analysed by gas-liquid chromatography.

The conversion of 3-pentenoic acid was 64% with a selectivity to ethylsuccinic anhydride of 95% and to 2-methylglutaric anhydride of 3%.

## EXAMPLE 2

An experiment was carried out as described in Example 1, charging the autoclave with the following materials:-

anisole 40 ml
3-pentenoic acid 10 ml (100 mmol)
palladium(II) acetate 0.4 mmol
1,4-di(diphenylphosphino)butane 1.2 mmol
tri(p-chlorophenyl)phosphine 3 mmol
p-toluenesulphonic acid 4 mmol

At the end of a reaction period of 1.5 h at 90 °C the conversion of 3-pentenoic acid was 96% with a selectivity to ethylsuccinic anhydride of 95%.

## EXAMPLE 3

An experiment was carried out as described in Example 2 with the exception that 1,4-di-(diphenylphosphino)butane was not present.

At the end of a reaction period of 1.5 h at 90 °C the conversion of 3-pentenoic acid was 100% with a selectivity to ethylsuccinic anhydride of 73% and to 2-methylglutaric anhydride of 26%.

Comparison with Example 2 shows that the presence of 1,4-di(diphenylphosphino)butane has increased the selectivity to ethylsuccinic anhydride.

## EXAMPLE 4

An experiment was carried out as described in Example 1, charging the autoclave with the following materials:-

anisole 40 ml
3-pentenoic acid 5 ml
palladium(II) acetate 0.4 mmol
triphenylphosphine 3 mmol
p-toluenesulphonic acid 3 mmol

At the end of a reaction period of 15 min at 90 °C the conversion of 3-pentenoic acid was 100% with a

selectivity to ethylsuccinic anhydride of 86% and to 2-methylglutaric anhydride of 14%.

EXAMPLE 5

An experiment was carried out as described in Example 1, charging the autoclave with the following materials:-
anisole 40 ml
3-pentenoic acid 5 ml
palladium(II) acetate 0.4 mmol
1,4-di(diphenylphosphino)butane 1.2 mmol
p-toluenesulphonic acid 4 mmol

At the end of a reaction period of 5 h at 90 °C the conversion of 3-pentenoic acid was 100% with a selectivity to ethylsuccinic anhydride of 100%.

Comparison with Example 2 shows that the presence of 1,4-di(diphenylphosphino)butane is sufficient to obtain a quantitative yield of ethylsuccinic anhydride.

EXAMPLE 6

Example 2 was repeated with the difference that anisole (40 ml) was replaced with sulfolane (40 ml).

At the end of a reaction period of 1.5 h at 90 °C the conversion of 3-pentenoic acid was 100% with a selectivity to ethylsuccinic anhydride of 95%.

EXAMPLE 7

An experiment was carried out as described in Example 1, charging the autoclave with the following materials:-
anisole 40 ml
crotonic acid 10 g
palladium(II) acetate 0.4 mmol
1,4-di(diphenylphosphino)butane 1.2 mmol
tri(p-chlorophenyl)phosphine 2 mmol
p-toluenesulphonic acid 3 mmol

At the end of a reaction period of 3.5 h at 150 °C the conversion of crotonic acid was 3% with a selectivity to methylsuccinic anhydride of 95%.

EXAMPLE 8

An experiment was carried out as described in Example 1, charging the autoclave with the following materials:-
anisole 40 ml
10-undecenoic acid 10 g
palladium(II) acetate 0.4 mmol
1,4-di(diphenylphosphino)butane 1.2 mmol
tri(p-chlorophenyl)phosphine 10 mmol
p-toluenesulphonic acid 4 mmol

At the end of a reaction period of 1.5 h at 145 °C the conversion of 10-undecenoic acid was 58% with a selectivity to octylsuccinic anhydride of 85%.

EXAMPLE 9

An experiment was carried out as described in Example 1, charging the autoclave with the following materials:-
sulfolane 40 ml
10-undecenoic acid 10 g
palladium(II) acetate 0.4 mmol
1,4-di(diphenylphosphino)butane 1.2 mmol
tri(p-chlorophenyl)phosphine 3 mmol
p-toluenesulphonic acid 4 mmol

At the end of a reaction period of 5 h at 145 °C the conversion of 10-undecenoic acid was 46% with a selectivity to octylsuccinic anhydride of 95%.

EXAMPLE 10

An experiment was carried out as described in Example 1, charging the autoclave with the following materials:-
anisole 40 ml
3-butenoic acid 10 ml (116 mmol)
palladium(II) acetate 0.4 mmol
1,4-di(diphenylphosphino)butane 1.2 mmol
tri(p-chlorophenyl)phosphine 2 mmol
p-toluenesulphonic acid 3 mmol

At the end of a reaction period of 0.5 h at 90 °C the conversion of 3-butenoic acid was 80% with a selectivity to methylsuccinic anhydride of 95%.

EXAMPLE 11

An experiment was carried out as described in Example 1, charging the autoclave with the following materials:
anisole 50 ml
oleic acid 10 ml
palladium(II) acetate 0.4 mmol
1,4-di(diphenylphosphino)butane 1.2 mmol
tri(p-chlorophenyl)phosphine 3 mmol
p-toluenesulphonic acid 4 mmol,
whereas the autoclave was heated to a temperature of 145 °C and kept at this temperature for 5 hours after pressurizing the autoclave with carbon monoxide until a pressure of 40 bar.

The conversion of oleic acid appeared to be 100%, with a selectivity to pentadecanyl succinic anhydride of more than 80% and the formation of some by-products due to the presence to traces of water.

## Claims

**1.** A process for the preparation of cyclic anhydrides of optionally substituted alkanedioic acids having 4 or 5 carbon atoms in the ring or of a mixture of such cyclic anhydrides which process comprises reacting an optionally substituted alkenoic acid with carbon monoxide in the presence of a catalytic system obtainable by combining the following components:-
- component (a) - a palladium compound,
- component (b) - a phosphine, arsine and/or stibine, and
- component (c) - a protonic acid having a $pK_a$, measured at 25 °C in aqueous solution, of less than 2.

**2.** A process as claimed in claim 1 in which the palladium compound is a palladium(II) salt of an alkanoic acid having not more than 12 carbon atoms per molecule.

**3.** A process as claimed in claim 1 or 2 in which component (b) is a chelating ligand comprising an organic compound containing as coordinating atoms at least two atoms of phosphorus, arsenic or antimony which are connected through a divalent organic bridging group having at least two carbon atoms in the bridge.

**4.** A process as claimed in claim 3 in which the chelating ligand has the general formula I

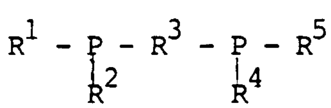

(I)

in which $R^1$, $R^2$, $R^4$ and $R^5$ represent identical or different optionally substituted hydrocarbon groups and $R^3$ represents a chain consisting of two to six optionally substituted methylene groups.

**5.** A process as claimed in claim 4 in which the chelating ligand is 1,4-di(diphenylphosphino)butane.

**6.** A process as claimed in claim 1 or 2 in which component (b) has the general formula II

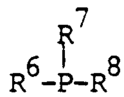

(II)

in which $R^6$, $R^7$ and $R^8$ each individually represent an optionally substituted aryl group.

**7.** A process as claimed in claim 6 in which the aryl groups are phenyl groups.

**8.** A process as claimed in any one of the preceding claims in which component (c) is an acid having the general formula III

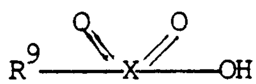

(III)

in which X represents a sulphur or a chlorine atom and, if X represents a chlorine atom, $R^9$ represents an oxygen atom and, if X represents a sulphur atom, $R^9$ represents an OH group or an optionally substituted hydrocarbon group.

**9.** A process as claimed in claim 8 in which component (c) is p-toluenesulphonic acid.

**10.** A process as claimed in any one of the preceding claims in which a molar ratio of component (a) to the optionally substituted alkenoic acid having a chain of at least 3 aliphatic carbon atoms in the range of from 1:10 to 1:10,000, a molar ratio of component (b) to component (a) and a molar ratio of component (c) to component (a) in the range of from 0.5 to 200 are used.

**11.** A process as claimed in any one of the preceding claims which is carried out at a temperature in the range of from 50 °C to 200 °C and at a pressure in the range of from 1 to 100 bar.

**12.** A process as claimed in any one of the preceding claims in which the alkenoic acid is a 3-alkenoic acid.

**13.** A process as claimed in claim 12 in which the 3-alkenoic acid is formed in situ by shifting of the carbon carbon double bond in a p-alkenoic acid in which p is an integer greater than 3.

## Revendications

**1.** Procédé de préparation d'anhydrides cycliques d'acides alcanedioÏques éventuellement substitués ayant 4 ou 5 atomes de carbone dans leur noyau ou d'un mélange de tels anhydrides cycliques, lequel procédé comprend la réaction d'un acide alcénoïque éventuellement substitué avec du monoxyde de carbone en présence d'un système catalytique que l'on peut obtenir en combinant les composants suivants :

| | |
|---|---|
| composant (a) - | composé de palladium |
| composant (b) - | phosphine, arsine et/ou stibine, et |
| composant (c) - | acide protonique ayant un $PK_a$ , mesuré à 25 °C en solution acqueuse, inférieur à 2 |

**2.** Procédé selon la revendication 1 dans lequel le composé de palladium est un sel de palladium(II) d'un acide alcanoïque n'ayant pas plus de 12 atomes de carbone par molécule.

**3.** Procédé selon la revendication 1 ou 2 dans lequel le composant (b) est un coordinat chélant comprenant un composé organique contenant comme atomes de coordination au moins deux atomes de phosphore, d'arsenic ou d'antimoine qui sont reliés par un groupe de pontage organique bivalent ayant au moins deux atomes de carbone dans le pont.

**4.** Procédé selon la revendication 3 dans lequel le coordinat chélant a la formule générale I

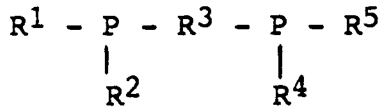

(I)

où $R^1$, $R^2$, $R^4$ et $R^5$ représentent des groupes hydrocarbonés éventuellement susbstitués identiques ou différents et $R^3$ représente une chaîne consistée de deux à six groupes méthylène éventuellement substitués.

**5.** Procédé selon la revendication 4 dans lequel le coordinat chélant est le 1,4 di(diphénylphosphino)-butane

**6.** Procédé selon la revendication 1 ou 2 dans laquelle le composant (b) a la formule générale II

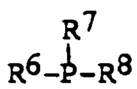

(II)

où $R^6$ , $R^7$ et $R^8$ représentent chacun individuellement un groupe aryle éventuellement substitué.

**7.** Procédé selon la revendication 6 dans lequel les groupes aryles sont des groupes phényles.

**8.** Procédé selon l'une quelconque des revendications précédentes dans lequel le composant (c) est un acide ayant la formule générale III

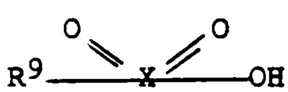

(III)

où X représente un atome de soufre ou de chlore, et si X représente un atome de chlore, $R^9$ représente un atome d'oxygène et, si X représente un atome de soufre, $R^9$ représente un groupe OH ou un groupe hydrocarboné éventuellement substitué.

**9.** Procédé selon la revendication 8 dans lequel le composant (c) est l'acide p-toluènesulfonique.

**10.** Procédé selon l'une quelconque des revendications précédentes dans lequel on utilise un rapport molaire du composant (a) à l'acide alcénoïque éventuellement substitué ayant une chaîne d'au moins 3 atomes de carbone aliphatique situé dans un intervalle de 1:10 à 1:10000, un rapport molaire du composant (b) au composant (a) et un rapport molaire du composant (c) au composant (a) situés dans un intervalle allant de 0,5 à 200.

**11.** Procédé selon l'une quelconque des revendications précédentes qui est réalisé a une température située dans un intervalle allant de 50%C à 200 °C et à une pression située dans un intervalle allant de 1 à 100 bars.

**12.** Procédé selon l'une quelconque des revendications précédentes dans lequel l'acide alcénoïque est l'acide 3-alcénoïque.

13. Procédé selon la revendication 12 dans lequel l'acide 3-alcénoïque est formé in situ en déplaçant la double liaison carbone-carbone dans un acide p-alcénoïque dans lequel p est un nombre entier supérieur à 3.

## Patentansprüche

1. Verfahren zur Herstellung von cyclischen Anhydriden von gegebenenfalls substituierten Alkandionsäuren mit 4 oder 5 Kohlenstoffatomen im Ring oder einem Gemisch solcher cyclischer Anhydride, wobei das Verfahren umfaßt die Umsetzung einer gegebenenfalls substituierten Alkensäure mit Kohlenmonoxid in Gegenwart eines katalytischen Systems, das erhältlich ist durch Zusammenbringen der folgenden Komponenten:
   Komponente (a) - eine Palladiumverbindung,
   Komponente (b) - ein Phosphin, Arsin und/oder Stibin und
   Komponente (c) - eine Protonsäure mit einem $pK_a$-Wert, gemessen bei 25 °C in wäßriger Lösung, von weniger als 2.

2. Verfahren nach Anspruch 1, wobei die Palladiumverbindung ein Palladium-(II)-Salz einer Alkansäure mit nicht mehr als 12 Kohlenstoffatomen im Molekül ist.

3. Verfahren nach Anspruch 1 oder 2, wobei die Komponente (b) ein chelatbildender Ligand ist, umfassend eine organische Verbindung, die als coordinierende Atome mindestens zwei Atome von Phosphor, Arsen oder Antimon enthält, die durch eine zweiwertige organische Brückengruppe mit mindestens zwei Kohlenstoffatomen in der Brücke miteinander verbunden sind.

4. Verfahren nach Anspruch 3, wobei der chelatbildende Ligand die allgemeine Formel I besitzt

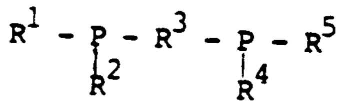

(I)

   in der $R^1$, $R^2$, $R^4$ und $R^5$ gleiche oder verschiedene, gegebenenfalls substituierte Kohlenwasserstoffgruppen bedeuten und $R^3$ eine aus zwei bis sechs, gegebenenfalls substituierten Methylengruppen bestehende Kette darstellt.

5. Verfahren nach Anspruch 4, wobei der chelatbildende Ligand 1,4-Di(diphenylphosphino)butan ist.

6. Verfahren nach Anspruch 1 oder 2, wobei die Komponente (b) die allgemeine Formel II besitzt

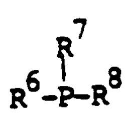

(II)

   in der $R^6$, $R^7$ und $R^8$ jeweils eine gegebenenfalls substituierte Arylgruppe bedeuten.

7. Verfahren nach Anspruch 6, wobei die Arylgruppen Phenylgruppen sind.

8. Verfahren nach einem der vorangehenden Ansprüche, wobei die Komponente (c) eine Säure ist der allgemeinen Formel III

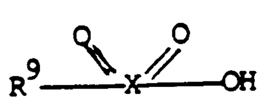

(III)

in der X ein Schwefel- oder Chloratom bedeutet und, wenn X ein Chloratom bedeutet, $R^9$ ein Sauerstoffatom ist und, wenn X ein Schwefelatom bedeutet, $R^9$ eine OH-Gruppe oder eine gegebenenfalls substituierte Kohlenwasserstoffgruppe ist.

**9.** Verfahren nach Anspruch 8, wobei die Komponente (c) p-Toluolsulfonsäure ist.

**10.** Verfahren nach einem der vorangehenden Ansprüche, wobei ein Molverhältnis von Komponente (a) zu der gegebenenfalls substituierten Alkensäure mit einer Kette von zumindest drei aliphatischen Kohlenstoffatomen im Bereich von 1:10 bis 1:10 000, ein Molverhältnis von Komponente (b) zu Komponente (a) und ein Molverhältnis von Komponente (c) zu Komponente (a) im Bereich von 0,5 bis 200 angewandt werden.

**11.** Verfahren nach einem der vorangehenden Ansprüche, das bei einer Temperatur im Bereich von 50°C bis 200°C und einem Druck im Bereich von 1 bis 100 bar durchgeführt wird.

**12.** Verfahren nach einem der vorangehenden Ansprüche, wobei die Alkensäure eine 3-Alkensäure ist.

**13.** Verfahren nach Anspruch 12, wobei die 3-Alkensäure in situ gebildet wird durch Verschiebung der Kohlenstoff-KohlenstoffDoppelbindung in einer p-Alkensäure, wobei p eine ganze Zahl großer 3 ist.